# EUROPEAN PATENT SPECIFICATION

(11) **EP 4 099 932 B1**
(45) Date of publication and mention of the grant of the patent: **03.04.2024**
(21) Application number: 21705074.9
(22) Date of filing: 21.01.2021
(51) Int. Cl.: A61B 18/18, A61B 34/20

(54) **SYSTEMS AND METHODS FOR MONITORING ABLATION ANTENNA MOVEMENT**
SYSTEME UND VERFAHREN ZUR ÜBERWACHUNG EINER ABLATIONSANTENNENBEWEGUNG
SYSTÈMES ET PROCÉDÉS DE SURVEILLANCE DE MOUVEMENT D'ANTENNE D'ABLATION

(30) Priority: 04.02.2020 US 202062969736 P
(43) Date of publication of application: 14.12.2022
(73) Proprietor: Covidien LP, Mansfield, MA 02048 (US)
(72) Inventor: BRANNAN, Joseph D., Boulder, Colorado 80301 (US); SOHLDEN, Ryan S., Boulder, Colorado 80301 (US)
(74) Representative: Maschio & Soames IP Ltd
(86) International application number: PCT/US2021/014308
(87) International publication number: WO 2021/158373

(56) References cited:
- EP-A1- 2 777 591
- EP-A1- 3 522 112
- US-A1- 2015 073 407
- US-A1- 2017 135 760
- US-A1- 2017 231 695

## Description

### FIELD

This disclosure provides a system and method for performing microwave ablation surgical treatment, and in particular, for monitoring movement of an ablation probe during the ablation treatment.

### BACKGROUND

Thermal ablation is used to coagulate diseased tissues, for example in solid organs such as the liver. To perform a thermal ablation, the user places a needle through a portion of the solid organ into the targeted diseased tissue. After placement, the needle is energized to heat the surrounding tissue to create an ablation zone encompassing the target. Precise placement of the needle with respect to the diseased tissue minimizes ablation of healthy tissue while ablating the entirety of the diseased tissue and a margin of tissue around the diseased tissue. After the ablation is created, the user will remove the needle from the organ, and optionally, heat the insertion tract during withdrawal of the needle. Movement of the needle following placement or during the procedure can cause imperfect ablation zones and unintended heating of other areas of the patient's anatomy. EP Patent Application EP 2777591 describes an ablation system with image database; US Patent Application 2017/135760 describes a system for ultra-sound image guided ablation antenna placement; US Patent Application 2017/231695 describes a system to determine the status of a microwave ablation system; US Patent Application 2015/073407 describes microwave ablation catheter systema and EP Patent Application EP 3522112 describes a system to map a lymphatic system using 3D modelling and lymph node mapping.

### SUMMARY

The disclosure provides a system and method for performing microwave ablation surgical treatment. In particular, the disclosure is directed to a system and method for filtering axial movement of an ablation probe from data relating to the position and orientation of the ablation probe before and during ablation of a target, and during tract ablation and withdrawal of the ablation probe from the patient.

According to an aspect of the disclosure, a system for performing a microwave ablation procedure includes an ablation probe, a tracking system for tracking a position and orientation of the ablation probe, and a computing device operably coupled to the tracking system and including a processor and a memory storing instructions which are executable by the processor. The computing device is configured to receive the position and orientation data of the ablation probe from the tracking system, display a graphical representation of the ablation probe on a display based on the received position and orientation data of the ablation probe, filter axial shift data from the position and orientation data of the ablation probe corresponding to axial movement of the ablation probe along a trajectory axis, and generate an alert based on the filtered axial shift data.

In an aspect, the alert is at least one of an audible alert or a visual alert.

In an aspect, the instructions cause the computing device to determine a movement velocity of the axial movement of the ablation probe along the trajectory axis based on the filtered axial shift data, and modulate the alert based on the determined movement velocity.

In an aspect, the instructions cause the computing device to determine a distance of the axial movement of the ablation probe along the trajectory axis from a baseline point on the trajectory axis based on the filtered axial shift data, and modulate the alert based on the determined distance.

In an aspect, the baseline point corresponds to a location of the ablation probe when an ablation procedure is initiated. Alternatively, the baseline point may be selectable by a user.

In accordance with another aspect of the disclosure, a method for monitoring movement of an ablation probe during a microwave ablation procedure includes tracking position and orientation data of the ablation probe in three-dimensional space, filtering axial shift data from the position and orientation data of the ablation probe, and generating an alert based on the filtered axial shift data. The filtered axial shift data corresponds to axial movement of the ablation probe along a trajectory axis.

In an aspect, generating the alert includes generating at least one of an audible alert or a visual alert.

In an aspect, the method includes determining a movement velocity of the axial movement of the ablation probe along the trajectory axis based on the filtered axial shift data, and modulating the alert based on the determined movement velocity.

In an aspect, the method includes determining a distance of the axial movement of the ablation probe along the trajectory axis from a baseline point on the trajectory axis based on the filtered axial shift data, and modulating the alert based on the determined distance.

In an aspect, the baseline point corresponds to a location of the ablation probe when an ablation procedure is initiated. Alternatively, the baseline point may be selectable by a user.

In accordance with another aspect of the disclosure, a non-transitory computer-readable storage medium stores instructions which, when executed by a processor, cause a computing device to receive position and orientation data of an ablation probe from a tracking system, display a graphical representation of the ablation probe on a display based on the received position and orientation data of the ablation probe, filter axial shift data from the position and orientation data of the ablation probe, and generate an alert based on the filtered axial shift data. The filtered axial shift data corresponds to axial movement of the ablation probe along a trajectory axis.

In an aspect, the alert is at least one of an audible alert or a visual alert.

In an aspect, the instructions cause the computing device to determine a movement velocity of the axial movement of the ablation probe along the trajectory axis based on the filtered axial shift data, and modulate the alert based on the determined movement velocity.

In an aspect, the instructions cause the computing device to determine a distance of the axial movement of the ablation probe along the trajectory axis from a baseline point on the trajectory axis based on the filtered axial shift data, and modulate the alert based on the determined distance.

In an aspect, the baseline point corresponds to a location of the ablation probe when an ablation procedure is initiated. Alternatively, the baseline point may be selectable by a user.

### BRIEF DESCRIPTION OF THE DRAWINGS

Objects and features of the presently disclosed system and method will become apparent to those of ordinary skill in the art when descriptions of various aspects thereof are read with reference to the accompanying drawings, of which:
FIG. 1 is a schematic diagram of a microwave ablation system in accordance with an aspect of the disclosure;
FIG. 2 is a schematic diagram of a computing device which forms part of the microwave ablation system of FIG. 1 in accordance with an aspect of the disclosure;
FIG. 3 is a flowchart illustrating a method for monitoring movement of an ablation probe in accordance with an aspect of the disclosure;
FIG. 4 is an example graphical user interface for enabling a movement detector in accordance with an aspect of the disclosure;
FIG. 5 is an example graphical user interface displaying a graphical representation of an ablation probe with a movement detector enabled in accordance with an aspect of the disclosure;
FIG. 6 is a flowchart illustrating a method for monitoring withdrawal of an ablation probe during a tract ablation procedure in accordance with an aspect of the disclosure;
FIG. 7A is an example graphical user interface displaying a graphical representation of an ablation probe during a monitored tract ablation procedure;
FIG. 7B is an example graphical user interface displaying a graphical representation of an ablation probe during a monitored tract ablation procedure;
FIG. 7C is an example graphical user interface displaying a graphical representation of an ablation probe during a monitored tract ablation procedure;
FIG. 8 is an example graphical user interface displaying a graphical representation of an ablation probe during a monitored tract ablation procedure;
FIG. 9A is an example graphical user interface displaying a graphical representation of an ablation probe during a monitored tract ablation procedure;
FIG. 9B is an example graphical user interface displaying a graphical representation of an ablation probe during a monitored tract ablation procedure; and
FIG. 9C is an example graphical user interface displaying a graphical representation of an ablation probe during a monitored tract ablation procedure.

### DETAILED DESCRIPTION

The disclosure provides a system and method for performing microwave ablation surgical treatment, and in particular, a method for monitoring movement of an ablation probe before, during, and after the ablation treatment.

In a first aspect, the system monitors movement of the ablation probe before, and/or during, application of ablation energy to the target, for the purpose of detecting and alerting a clinician of unwanted (or undesired) movement of the ablation probe. During an ablation procedure, an ablation probe is navigated to a treatment target and positioned in (or proximate) the target for a certain period of time (e.g., five minutes) during application of microwave energy. During this time, the system monitors movement of the probe to alert the clinician of any movement of the ablation probe. Because the ablation probe may move relative to a 3D space, but still remain fixed in position relative to the target (for example, during patient breathing where both the target and the ablation probe move synchronously), the disclosed system filters the axial movement of the ablation probe along the probe's axis and only alerts the clinician to axial movement of the ablation probe. Several user interfaces and modulated audible and visual alerts are disclosed for assisting the clinician in ensuring that the ablation probe does not experience any undesired or unwanted axial movement before or during the ablation procedure.

As described in detail below, the ablation probe position and orientation are known with respect to an electromagnetic field generator. The disclosed computing device filters that position and orientation data to track the change in the axial shift of the ablation probe position. This filtered parameter informs on the change in position in time of the ablation probe within the patient along the direction most likely to move unintentionally. By filtering on axial shift only, reduction in sources of noise such as from patient breathing would be accomplished. Optionally, separate position tracking sensors could be placed on the patient to track patient movement more precisely to further improve the isolation of antenna shaft movement relative to the patient anatomy.

In a second aspect, the system monitors axial movement of the ablation probe during a tract ablation procedure, during which time the clinician is withdrawing the ablation probe from the patient and applying ablation energy to the tissue tract created by the ablation needle, thereby providing the clinician with insight into whether the clinician is retracting the ablation probe at the correct rate. Several user interfaces and modulated audible and visual alerts are disclosed for assisting the clinician in withdrawing the ablation probe at an appropriate rate.

Liver tissue is highly vascularized and may bleed along the tract of the ablation needle between the ablation zone and the organ boundary, particularly if a blood vessel was pierced by the needle. To mitigate risk of a bleeding needle tract, users will coagulate the tract using the energized ablation needle. Currently, to do this successfully (minimize over-ablation of healthy tissue along the tract while ensuring full coagulation of the tract), the user must understand a complex relationship between the power delivered to the ablation needle and the retraction rate of the ablation needle. The second aspect of this disclosure reduces the complexity of tract ablation for users, simplifying the intraprocedural task of successfully coagulating a needle tract without over-ablating surrounding healthy tissue. Many patients who undergo ablation in the liver have degraded liver function and therefore it is important to preserve as much functional liver tissue as possible.

Although the disclosure will be described in terms of specific illustrative aspects, it will be readily apparent to those skilled in this art that various modifications, rearrangements and substitutions may be made. The scope of the invention is defined by the claims appended hereto. As used herein, the term "clinician" refers to any medical professional (e.g., doctor, surgeon, nurse, or the like) or other user of the system involved in planning, performing, monitoring and/or supervising a medical procedure involving the use of the systems and methods described herein.

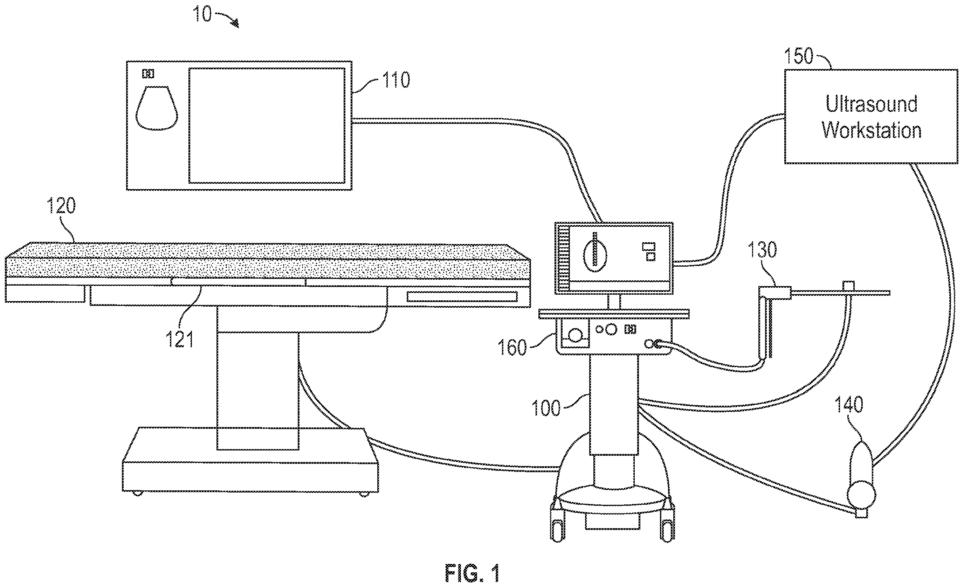

FIG. 1 illustrates a treatment system 10, which includes a computing device 100, a display 110, a table 120, an ablation probe 130, an ultrasound imager 140, an ultrasound workstation 150, and a generator 160. Computing device 100 may be, for example, a laptop computer, desktop computer, tablet computer, or other similar device. Computing device 100 may be configured to control an electrosurgical generator, a peristaltic pump, a power supply, and/or any other accessories and peripheral devices relating to, or forming part of, system 10. Although ultrasound workstation 150 is illustrated in FIG. 1 as a separate component from computing device 100, in aspects, ultrasound workstation 150 may be incorporated into computing device 100 with user interfaces displaying data of ultrasound workstation 150 on a display of computing device 100.

Display 110 is configured to output instructions, images, and messages relating to the performance of the microwave ablation procedure in the form of the graphical user interfaces described below. Although display 110 is shown as a separate component from computing device 100, in aspects, display 110 is a component of computing device 100, where computing device 100 includes one or more displays for displaying various user interfaces displaying data corresponding to ultrasound data and navigation and ablation parameters and data. Table 120 may be, for example, an operating table or other table suitable for use during a surgical procedure, which includes an electromagnetic (EM) field generator 121. EM field generator 121 is used to generate an EM field during the microwave ablation procedure and forms part of an EM tracking system which is used to track the positions of surgical instruments, such as ablation probe 130 and ultrasound imager 140, within and around the body of a patient. The EM tracking system (or another tracking system) may additionally include sensors for tracking movement of the patient (e.g., breathing) and may utilize such patient tracking movement to compensate for any displayed elements. Such sensors may include one or more electromagnetic tracking sensors positionable on a patient's chest, which tracks patient body movement independently from any other device (ultrasound wand 140 or ablation probe 130) movements.

Ablation probe 130 is a surgical instrument having a microwave ablation antenna which is used to ablate tissue. In particular, ablation probe 130 may be used to ablate tissue, such as a lesion or tumor (hereinafter referred to as a "target") by using electromagnetic radiation or microwave energy to heat tissue in order to denature or kill cells, e.g., cancerous cells. The location of ablation probe 130 within the body of the patient may be tracked during the surgical procedure. An example method of tracking the location of ablation probe 130 is by using the EM tracking system, which tracks the location of ablation probe 130 by tracking sensors attached to or incorporated in ablation probe 130.

In addition to the EM tracking system, the surgical instruments may also be visualized by using ultrasound imaging. Ultrasound imager 140, such as an ultrasound wand, may be used to image the patient's body during the microwave ablation procedure to visualize the location of the surgical instruments, such as ablation probe 130, inside the patient's body along with the anatomy of the patient. Ultrasound imager 140 may have an EM tracking sensor embedded within or attached to the ultrasound wand, for example, a clip-on sensor or a sticker sensor. As described further below, ultrasound imager 140 may be positioned in relation to ablation probe 130 such that ablation probe 130 is at an angle to the ultrasound image plane, thereby enabling the clinician to visualize the spatial relationship of ablation probe 130 with the ultrasound image plane and with objects being imaged.

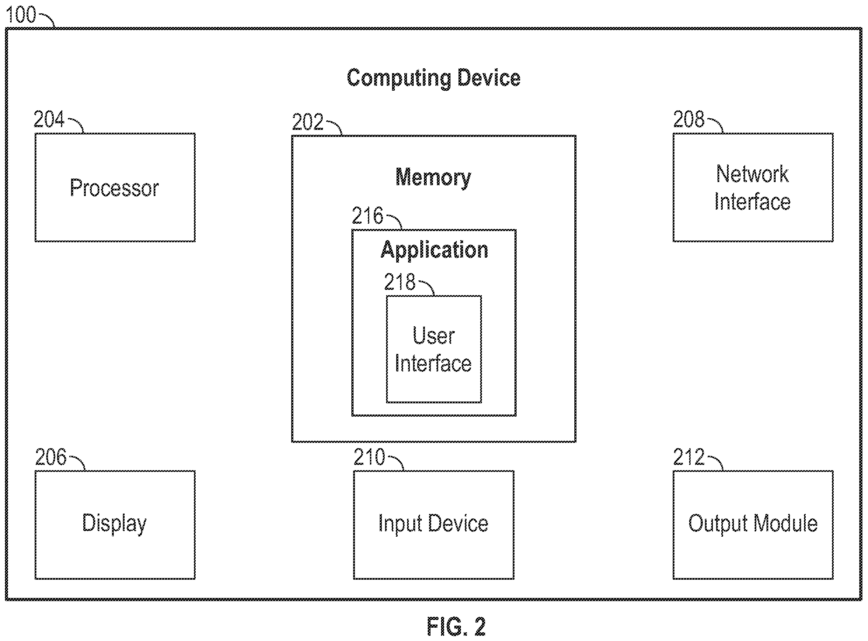

Turning now to FIG. 2, there is shown a system diagram of computing device 100. Computing device 100 may include memory 202, processor 204, display 206, network interface 208, input device 210, and/or output module 212.

Memory 202 includes any non-transitory computer-readable storage media for storing data and/or software that is executable by processor 204 and which controls the operation of computing device 100. In an aspect, memory 202 may include one or more solid-state storage devices such as flash memory chips. Although the description of computer-readable media contained herein refers to a solid-state storage, it should be appreciated by those skilled in the art that computer-readable storage media can be any available media that can be accessed by the processor 204. That is, computer readable storage media includes non-transitory, volatile and non-volatile, removable and non-removable media implemented in any method or technology for storage of information such as computer-readable instructions, data structures, program modules or other data. Memory 202 may store application 216 which may, when executed by processor 204, cause display 206 to present user interface 218 and perform any or all of the steps of the methods described herein.

Processor 204 may be a general purpose processor, a specialized graphics processing unit (GPU) configured to perform specific graphics processing tasks while freeing up the general purpose processor to perform other tasks, and/or any number or combination of such processors.

Display 206 may be touch sensitive and/or voice activated, enabling display 206 to serve as both an input and output device. Alternatively, a keyboard (not shown), mouse (not shown), or other data input devices may be employed.

Network interface 208 may be configured to connect to a network such as a local area network (LAN) consisting of a wired network and/or a wireless network, a wide area network (WAN), a wireless mobile network, a Bluetooth network, and/or the internet. Input device 210 may be any device by means of which a user may interact with computing device 100, such as, for example, a mouse, keyboard, foot pedal, touch screen, and/or voice interface. Output module 212 may include any connectivity port or bus, such as, for example, parallel ports, serial ports, universal serial busses (USB), or any other similar connectivity port known to those skilled in the art.

Application 216 may be one or more software programs stored in memory 202 and executed by processor 204 of computing device 100. As will be described in more detail below, during the planning phase, application 216 guides a clinician through a series of steps to identify a target, size the target, size a treatment zone, and/or determine an access route to the target for later use during the procedure phase. In some aspects, application 216 is loaded on computing devices in an operating room or other facility where surgical procedures are performed, and is used as a plan or map to guide a clinician performing a surgical procedure, but without any feedback from ablation probe 130 used in the procedure to indicate where ablation probe 130 is located in relation to the plan. In other aspects, system 10 provides computing device 100 with data regarding the location of ablation probe 130 within the body of the patient, such as by EM tracking, which application 216 may then use to indicate on the plan where ablation probe 130 are located.

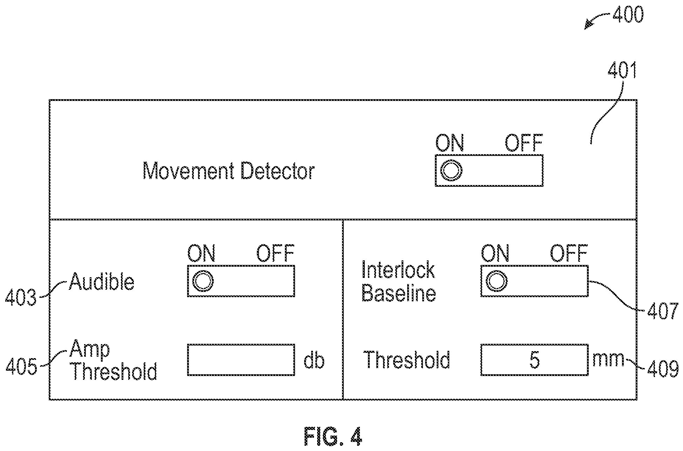

Application 216 communicates with a user interface 218 which generates a user interface for presenting visual interactive features to a clinician, for example, on display 206 and for receiving clinician input, for example, via a user input device. For example, user interface 218 may generate a graphical user interface (GUI) and output the GUI to display 206 for viewing by a clinician. Examples of the GUI are described below with reference to FIGS. 4, 5, 7A-7C, 8, and 9A-9C.

Computing device 100 is linked to display 110, thus enabling computing device 100 to control the output on display 110 along with the output on display 206. Computing device 100 may control display 110 to display output which is the same as or similar to the output displayed on display 206. For example, the output on display 206 may be mirrored on display 110. Alternatively, computing device 100 may control display 110 to display different output from that displayed on display 206. For example, display 110 may be controlled to display guidance images and information during the microwave ablation procedure, while display 206 is controlled to display other output, such as configuration or status information.

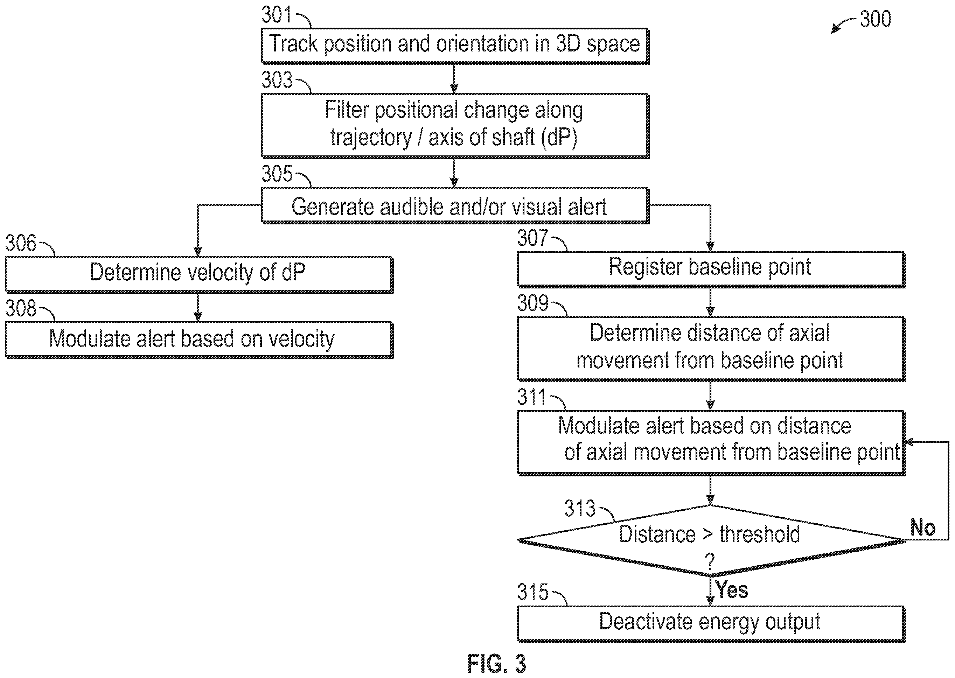

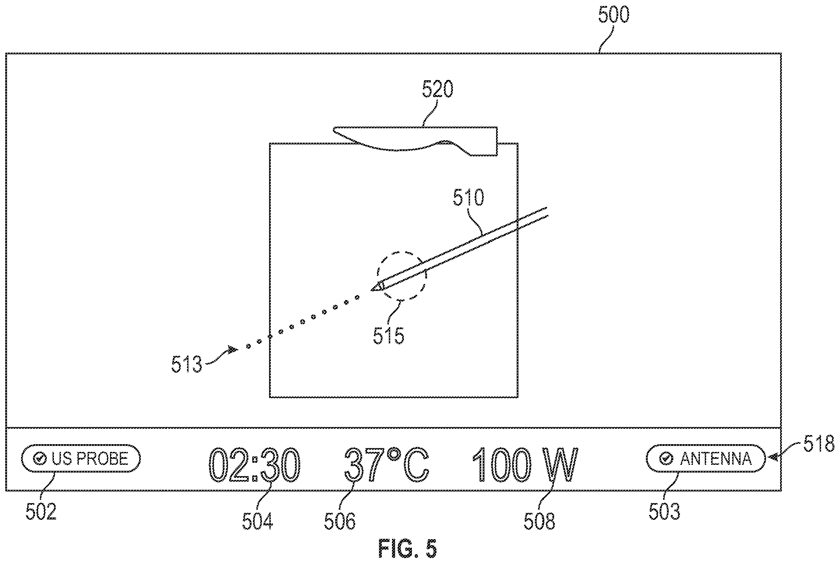

FIG. 3 illustrates a flowchart for a method for monitoring a position of an ablation probe, and alerting a user of undesired movement of the ablation probe, and is described as method 300. Method 300 begins at step 301 where a position and orientation of an ablation probe is tracked. Such tracking may be achieved by an electromagnetic tracking system as described above. Referring briefly to FIG. 5, the computing device 100 may display a user interface 500 including a graphical representation of the ablation probe 510 relative to an ultrasound plane 520 in 3D space, with the graphical representation of the ablation probe 510 and the ultrasound plane 520 moving relative to each other based on their corresponding tracked positions and orientations. In step 303, the computing device 100 filters the positional change of the ablation probe along its trajectory (e.g., along an axis of the ablation probe's shaft). In particular, in step 303, the computing device 100 filters axial shift data (dP) from the position and orientation data of the ablation probe. The filtered axial shift data corresponds to axial movement of the ablation probe along its trajectory axis, and discounts and ignores any movement of the ablation probe along any other axis.

In step 305, computing device 100 generates an alert based on the filtered axial shift data. The alert generated in step 305 may be minimal in the event that no actual shift of the ablation probe has yet occurred. The alert generated in step 305 is configured to be modulated, as described in the steps below, and may include one or any of a tactile, audible, or visual alert.

In step 306, computing device 100 determines a movement velocity of the axial movement of the ablation probe along the trajectory axis based on the filtered axial shift data and, in step 308, modulates the generated alert based on the determined movement velocity. The movement velocity (dx, dy, dz/dt) may be multiplied by a customizable amplitude for modulating the generated alert. In use, the clinician may be presented with a continuous audible alert that corresponds to the velocity of the movement of the probe. For example, where the velocity of axial movement is slow, the audible tone may be low in volume and where the velocity of axial movement is fast, the audible tone may be loud in volume to alert the user of such drastic movement along the trajectory. As the modulation of the alert (e.g., audible output) is based on the filtered axial shift data, any tracked movement of the probe that is non-axial is not accounted for and does not affect the modulation of the alert. Therefore, even if the non-axial velocity of movement of the probe is fast (for example, due to patient breathing or an abrupt cough), such a high velocity movement of the probe in a non-axial direction does not cause any change in the modulation of the alert.

In step 307, computing device 100 registers a baseline point along the probe's trajectory. The baseline point may be selected by a user or may be automatically assigned as corresponding to the axial position of the probe when microwave ablation begins. In step 309, computing device 100 determines a distance of the axial movement of the ablation probe along the trajectory axis from the baseline point on the trajectory axis based on the filtered axial shift data, and in step 311, modulates the alert based on the determined distance from the baseline point. In use, the computing device 100 outputs a continuous alert (e.g., audible alert) which alerts the clinician as to how far the ablation probe has moved along the trajectory from the point where the ablation probe was located when ablation first initiated. As the ablation probe moves further away, along its trajectory, from the initial position where ablation began, the alert is modulated greater (e.g., the audible alert is louder). As the modulation of the alert (e.g., audible output) is based on the filtered axial shift data, any tracked movement of the probe that is non-axial is not accounted for and does not affect the modulation of the alert. Therefore, even if the non-axial distance of movement of the probe is great (for example, due to patient breathing or an abrupt cough), such a movement of the probe in a non-axial direction does not cause any change in the modulation of the alert.

In step 313, the computing device 100 determines if the distance from the baseline point (determined in step 309) is greater than a threshold distance. The threshold distance may be input by a user or determined by the computing device 100 based on the location of the target, the size of the target, and/or the organ being treated. If the distance exceeds the threshold, then computing device 100 deactivates, or reduces, the output of microwave energy. Such an indication of axial movement away from the target is indicative that the ablation probe may not be position in a suitable area to be ablating the target, and may instead by ablating healthy tissue. Alternatively, if it is determined that the determined distance does not exceed the threshold, then the computing device 100 continues to output the ablation energy.

FIG. 4 illustrates an example user interface 400 of computing device 100 which displays activation and configuration settings for controlling the method for monitoring a position of an ablation probe, and alerting a user of undesired movement of the ablation probe, (e.g., method 300). User interface 400 includes an activation switch 401 for switching the feature on and off. The alerts generated in method 300 may be set as audible alerts by selector 403 of user interface 400. Additionally, the customizable amplitude described above, which may be multiplied by the movement velocity (dx, dy, dz/dt), is input by a user, or the display of such amplitude when calculated by computing device 100, in section 405 of user interface 400. The baseline interlock feature described above in steps 307-315 of method 300 is activated or deactivated by selector 407 of user interface 400. Finally, the threshold distance, as selected by a user or as calculated by computing device 100, which is described above in step 313, is displayed in section 409 of user interface 400.

FIG. 5 illustrates an example user interface 500 which displays a graphical representation of the tracked ablation probe 510 relative to an ultrasound plane 520 in 3D space. The ablation probe's trajectory 513 is displayed as extending longitudinally parallel to the longitudinal axis of the ablation probe. An ablation zone 515 is displayed, which corresponds to the displayed settings in settings section 518 of the electrosurgical generator to which the ablation probe is coupled.

Ultrasound plane 520 will include an ultrasound image (not shown here for the purpose of more clearly depicting the elements being described) based on ultrasound image data captured by ultrasound imager 140 (FIG. 1). User interface 500 further includes an ultrasound probe indicator 502 and antenna indicator 503 that indicates whether ultrasound imager 140 and ablation probe 130 are connected to computing device 100 and system 10. In the settings section 518 of user interface 500, indicators of a time 504, temperature 506, and wattage 508 configured for the current ablation procedure are also displayed.

Trajectory 513 shows the trajectory at which ablation probe 130 is being navigated inside the patient's body. The length of trajectory 513 corresponds to the length of ablation probe 130. Likewise, the width of trajectory 513 corresponds to the width of ablation probe 130. Thus, when positioning ablation probe 130 and ultrasound imager 140 outside the patient's body, trajectory 513 will show the distance ablation probe 130 can be navigated into the patient's body. As such, the clinician can determine whether ablation probe 130 can reach the target tissue inside the patient's body before inserting ablation probe 130 into the patient's body.

User interface 500 may depict the graphical representation of ablation probe 510 and trajectory 513 as outlines, such that the ultrasound image displayed on ultrasound plane 520 is not obscured by the graphical representation of ablation probe 510 and trajectory 513.

In accordance with the method 300 (FIG. 3) described above and the example user interfaces 400 (FIG. 4), 500 (FIG. 5) associated therewith, the computing device 100 generates and modulates an alert based on the axial movement of the ablation probe along its longitudinal axis. By alerting a clinician as to the axial movement of the ablation probe, before or during a microwave ablation procedure, the clinician is continuously made aware of the ablation probe's position relative to the target it is treating. In other words, movement of the ablation probe in a non-axial direction, as caused by patient movement (e.g., breathing), is considered by the computing device 100 to be consistent with movement of the target as well, and therefore, is not problematic and not worthy of the clinician's attention.

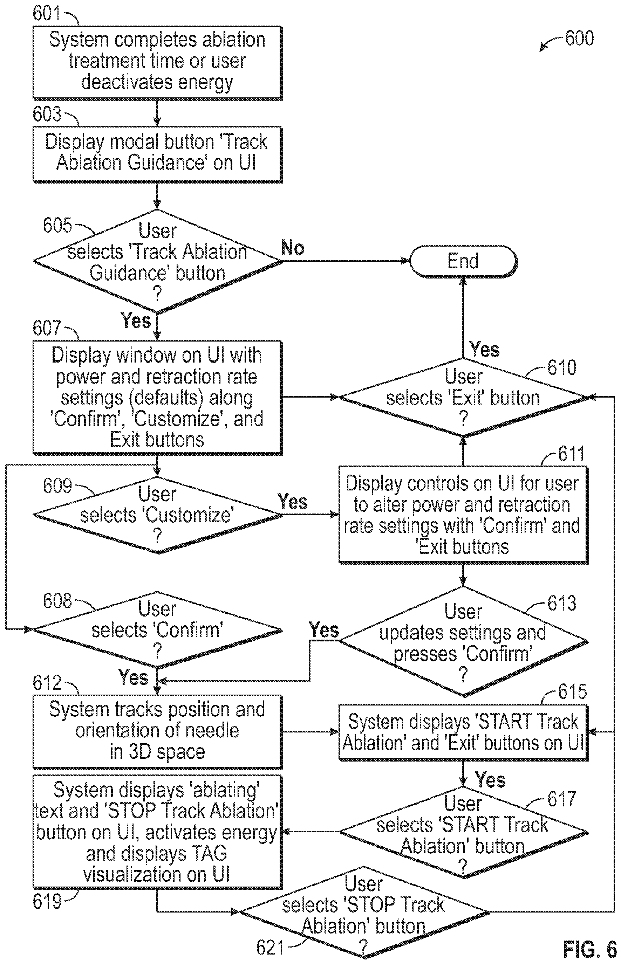

FIG. 6 illustrates a flowchart of a method for guiding a tract ablation procedure and is described as method 600. In particular, method 600 is directed to monitoring the withdrawal rate of the ablation probe during tract ablation and informing the clinician as to whether the actual rate of withdrawal is too fast or too slow relative to a recommended rate of withdrawal. Like method 300, described above, method 600 also filters the axial movement of the ablation probe along is trajectory, and does not consider any movement of the ablation probe in a non-axial direction.

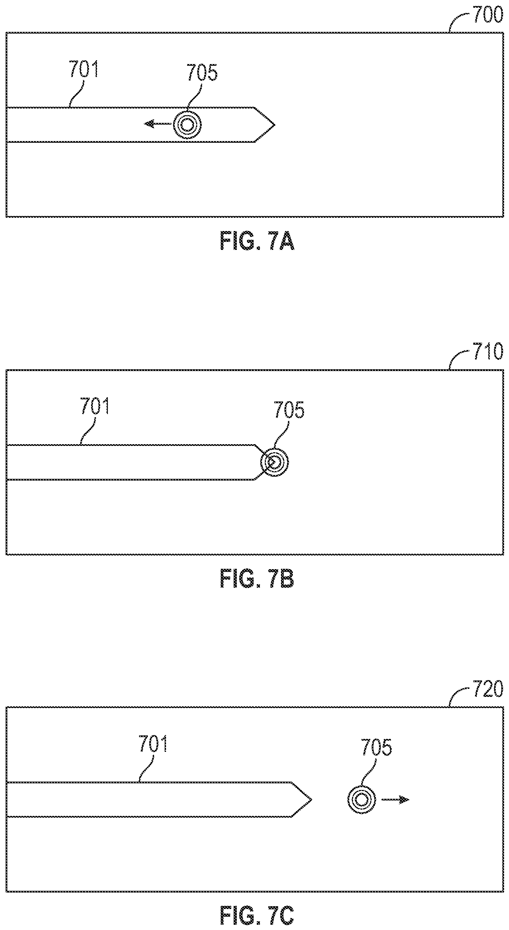

After ablation of the target is complete (step 601), as energy is deactivated by a user or the treatment time is complete, a "tract ablation guidance" button is displayed (step 603) for optional selection by a clinician. If the clinician selects to track the tract ablation procedure (yes in step 605), then in step 607 a window is displayed with the power and retraction rate settings, with the option to confirm or customize the recommended settings. If the clinician selects to customize (yes in step 609), the in step 611 the user is presented with a user interface for altering the power and/or time setting for the tract ablation procedure with "confirm" and "exit" buttons. Upon selecting the recommended settings in either of steps 608 or 613, in step 612, the computing device 100 tracks the position and orientation of the ablation probe in 3D space. Step 612 may additionally include filtering the axial position data of the position and orientation of the ablation probe, in particular the position and orientation data of the ablation probe along the longitudinal axis of the probe. In step 615, "start tract ablation" and "exit" buttons are displayed. When a clinician selects to start the tract ablation (yes in step 617), the computing device 100 commands the electrosurgical generator to begin outputting the ablation energy and displays the tract ablation guidance user interfaces (e.g., FIGS. 7A-7C, 8, and 9A-9C) for guiding the clinician throughout the tract ablation procedure. Upon completion, or before completion if desired by the clinician, the clinician may select the "stop tract ablation" button displayed in step 621.

FIGS. 7A-7C illustrate user interfaces 700, 710, 720 displayable by the system during a tract ablation procedure that assist a clinician in visualizing the actual rate of withdrawal relative to a recommended rate of withdrawal. In particular, user interface 700 (FIG. 7A) illustrates a graphical representation of an ablation probe 701 relative to a marker 705 when the actual withdrawal rate of the ablation probe is slower than the recommended rate of withdrawal; user interface 710 (FIG. 7B) illustrates a graphical representation of an ablation probe 701 relative to a marker 705 when the actual withdrawal rate of the ablation probe is consistent with the recommended rate of withdrawal; and user interface 720 (FIG. 7C) illustrates a graphical representation of an ablation probe 701 relative to a marker 705 when the actual withdrawal rate of the ablation probe is faster than the recommended rate of withdrawal. In each of user interfaces 700, 710, 720, the graphical representation of the ablation probe 701 is fixed in position and marker 705 moves relative thereto.

During use, the marker 705 is initially oriented over an image of the tip of the graphical representation of the ablation probe 701. The marker 705 moves in relation to the needle tip depending upon the configured/recommended retraction rate and the actual retraction rate of the ablation probe. If the actual retraction rate of the ablation probe is too slow relative to the configured retraction rate, the ball will drift proximally along the graphical representation of the ablation probe 701 (proximally away from the needle tip), transitioning from the user interface 710 to the user interface 700. Conversely, if the actual retraction rate of the ablation probe is too fast relative to the configured retraction rate, the ball will drift distally along the graphical representation of the ablation probe 701 (distally away from the needle tip), transitioning from the user interface 710 to the user interface 720. The clinician intends to keep the marker 705 directly over the needle tip of the graphical representation of the ablation probe 701 to most closely resemble the user interface 710. If the marker 705 drifts outside of given distance range of the tip (such as 1.0 cm), the marker 705 disappears and then reappears on the needle tip of the ablation probe. Additional arrows could be used to clarify the ideal location of the marker 705. Additionally, the orientation of the graphical representation of the ablation probe 701 within the user interfaces 700, 710, 720 could updated based upon actual orientation of ablation probe so the correct perspective is provided to the clinician.

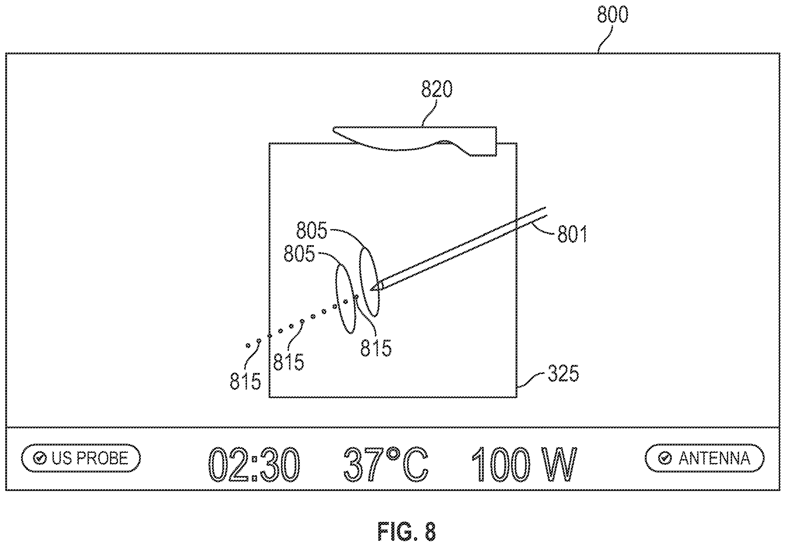

FIG. 8 illustrates a user interface 800 displayable by the system during a tract ablation procedure that assist a clinician in visualizing the actual rate of withdrawal relative to a recommended/preconfigured rate of withdrawal. User interface 800 includes a graphical representation of the ablation probe 801 relative to trajectory rings 815 and guidance markers 805. The retraction rate guidance markers 805 are displayed, fixed in position, relative to the moving graphical representation of the ablation probe 801. Specifically, the markers 805 are axially locked to trajectory rings 815 but are displayed larger, bolder, and/or a different color. The guidance marker 805 is initially oriented over the needle tip of the graphical representation of the ablation probe 801. The guidance marker 805 moves in relation to the needle tip depending upon the configured/recommended retraction rate and the actual retraction rate of the ablation probe. If the actual retraction rate of the ablation probe is too slow relative to the configured retraction rate, the marker 805 will drift proximally along the displayed graphical representation of the ablation probe 801. Conversely, if the actual retraction rate of the ablation probe is too fast relative to the configured retraction rate, the marker 805 will drift distally along the displayed graphical representation of the ablation probe 801. The clinician intends to keep the marker 805 directly over the needle tip of the graphical representation of the ablation probe 801, indicating that the actual retraction rate equals the configured/recommended retraction rate. If the marker 805 drifts outside of given distance range of the tip (such as 1.0 cm) the marker 805 disappears and then reappears on the needle tip. Although illustrated marker 805 is illustrated as a ring, marker 805 may alternatively, or additionally, include a bar.

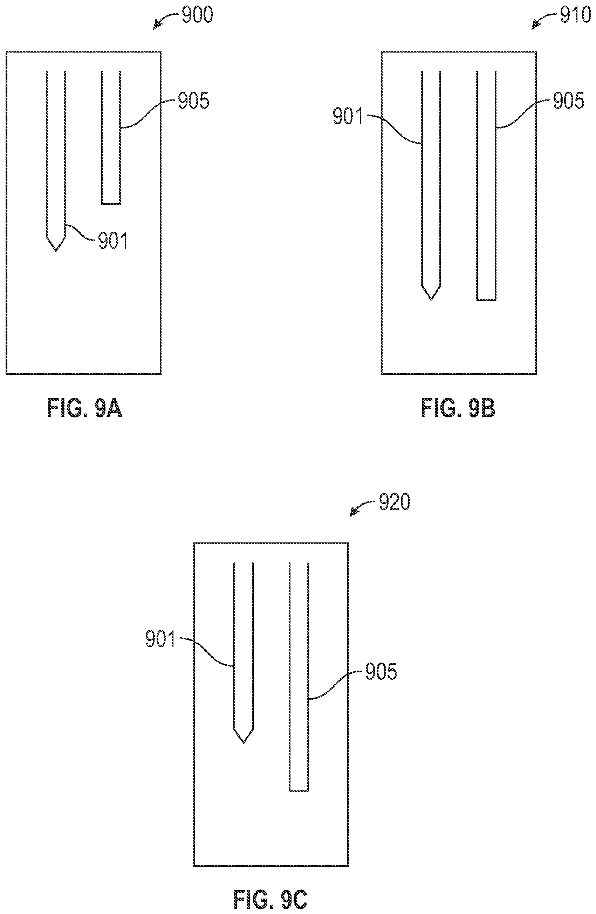

FIGS. 9A-9C illustrate other user interfaces 900, 910, 920 displayable by the system during a tract ablation procedure that assist a clinician in visualizing the actual rate of withdrawal relative to a recommended/preconfigured rate of withdrawal. User interfaces 900, 910, 920 include a display of an optimal ablation probe marker 905 and an ablation probe position marker 901, each of which being movable relative to the other. In user interface 910, the optimal ablation probe marker 905 for the configured/recommended retraction rate is initially oriented in line with the ablation probe position marker 901. The ablation probe markers 901, 905 move in relation to one another depending upon the configured/recommended retraction rate and the actual retraction rate of the ablation probe. If the retraction rate of the ablation probe is too slow relative to the configured/recommended retraction rate, the optimal ablation probe marker 905 will pull ahead of the actual ablation probe position marker 901 (FIG. 9A). Conversely, if the retraction rate of the ablation probe is too fast relative to the configured/recommended retraction rate, the optimal ablation probe marker 905 will fall behind the actual ablation probe position marker 901 (FIG. 9C). The clinician intends to keep the display of an optimal ablation probe marker 905 and an ablation probe position marker 901 in-line with each other. If the actual ablation probe position marker 901 drifts away from the optimal ablation probe marker 905, or vice-versa, by a given distance range (such as 1.0cm) the optimal ablation probe marker 905 disappears and then reappears in line with the ablation probe position marker 901. The user interfaces 900, 910, 920 can also be displayed in the 3D space alongside the ghost image of a graphical representation of an ablation device.

Although aspects have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing aspects may be made.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed, entirely or partially, by a single module or unit for purposes of clarity (e.g., computing device 100), it should be understood that the techniques of this disclosure may be performed by a combination of units or modules associated with, for example, a medical device, in full or in part (e.g., other components of system 10).

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A system (10) for performing a microwave ablation procedure, the system comprising:
an ablation probe (130) configured to couple to an electrosurgical generator and including an electromagnetic sensor;
a tracking system configured to track movement of the ablation probe in three-dimensional space and generate position and orientation data of the ablation probe based on a position and orientation of the electromagnetic sensor; and
a computing device (100) operably coupled to the tracking system, the computing device including a processor (204) and a memory (202) storing instructions which, when executed by the processor, cause the computing device to:
receive the position and orientation data of the ablation probe from the tracking system;
display a graphical representation of the ablation probe on a display (110)
based on the received position and orientation data of the ablation probe; **characterised in that** said instructions, when executed by the processor, additionally cause the computing device to:
filter axial shift data from the position and orientation data of the ablation probe, the filtered axial shift data corresponding to axial movement of the ablation probe along a trajectory axis; and
generate an alert based on the filtered axial shift data.

2. The system of claim 1, wherein the alert is at least one of an audible alert or a visual alert.

3. The system of claim 1 or claim 2, wherein the instructions further cause the computing device to:
determine a movement velocity of the axial movement of the ablation probe along the trajectory axis based on the filtered axial shift data; and
modulate the alert based on the determined movement velocity.

4. The system of any preceding claim, wherein the instructions further cause the computing device to:
determine a distance of the axial movement of the ablation probe along the trajectory axis from a baseline point on the trajectory axis based on the filtered axial shift data; and
modulate the alert based on the determined distance.

5. The system of claim 4, wherein the baseline point corresponds to a location of the ablation probe when an ablation procedure is initiated; or wherein the baseline point is selectable by a user.

6. A non-transitory computer-readable storage medium storing instructions which, when executed by a processor, cause a computing device to:
receive position and orientation data of an ablation probe (130) from a tracking system;
display a graphical representation of the ablation probe on a display (110) based on the received position and orientation data of the ablation probe;
**characterised in that** said instructions, when executed by the processor, additionally cause the computing device to:
filter axial shift data from the position and orientation data of the ablation probe, the filtered axial shift data corresponding to axial movement of the ablation probe along a trajectory axis; and
generate an alert based on the filtered axial shift data.

7. The non-transitory computer-readable storage medium of claim 6, wherein the alert is at least one of an audible alert or a visual alert.

8. The non-transitory computer-readable storage medium of claim 6 or claim 7, wherein the instructions further cause the computing device to:
determine a movement velocity of the axial movement of the ablation probe along the trajectory axis based on the filtered axial shift data; and
modulate the alert based on the determined movement velocity.

9. The non-transitory computer-readable storage medium of any of claims 6 to 8, wherein the instructions further cause the computing device to:
determine a distance of the axial movement of the ablation probe along the trajectory axis from a baseline point on the trajectory axis based on the filtered axial shift data; and
modulate the alert based on the determined distance.

10. The non-transitory computer-readable storage medium of claim 9, wherein the baseline point corresponds to a location of the ablation probe when an ablation procedure is initiated.

11. The non-transitory computer-readable storage medium of claim 9, wherein the baseline point is selectable by a user.

## Patentansprüche

1. System (10) zum Durchführen einer Mikrowellenablationsprozedur, wobei das System umfasst:
eine Ablationssonde (130), die dafür ausgelegt ist, an einen elektrochirurgischen Generator gekoppelt zu werden, und die einen elektromagnetischen Sensor aufweist;
ein Verfolgungssystem, das dafür ausgelegt ist, eine Bewegung der Ablationssonde im dreidimensionalen Raum zu verfolgen und Positions- und Ausrichtungsdaten der Ablationssonde zu generieren, basierend auf einer Position und Ausrichtung des elektromagnetischen Sensors; und
eine Rechenvorrichtung (100), die operativ an das Verfolgungssystem gekoppelt ist, wobei die Rechenvorrichtung einen Prozessor (204) und einen Speicher (202) aufweist, welcher Anweisungen speichert, die, wenn sie von dem Prozessor ausgeführt werden, die Rechenvorrichtung hierzu veranlassen:
Empfangen der Positions- und Ausrichtungsdaten der Ablationssonde von dem Verfolgungssystem;
Anzeigen einer grafischen Darstellung der Ablationssonde auf einer Anzeige (110), basierend auf den empfangenen Positions- und Ausrichtungsdaten der Ablationssonde;
**dadurch gekennzeichnet, dass** die Anweisungen, wenn sie von dem Prozessor ausgeführt werden, den Prozessor zusätzlich hierzu veranlassen:
Filtern von Axialverschiebungsdaten aus den Positions- und Ausrichtungsdaten der Ablationssonde, wobei die gefilterten Axialverschiebungsdaten einer axialen Bewegung der Ablationssonde entlang einer Trajektorieachse entsprechen; und
Generieren eines Alarms basierend auf den gefilterten Axialverschiebungsdaten.

2. System nach Anspruch 1, wobei der Alarm wenigstens eines von einem akustischen Alarm oder einem visuellen Alarm ist.

3. System nach Anspruch 1 oder Anspruch 2, wobei die Anweisungen die Rechenvorrichtung ferner hierzu veranlassen:
Bestimmen einer Bewegungsgeschwindigkeit der Axialbewegung der Ablationssonde entlang der Trajektorieachse basierend auf den gefilterten Axialverschiebungsdaten; und
Modulieren des Alarms basierend auf der bestimmten Bewegungsgeschwindigkeit.

4. System nach einem der vorstehenden Ansprüche, wobei die Anweisungen die Rechenvorrichtung ferner hierzu veranlassen:
Bestimmen eines Abstands der Axialbewegung der Ablationssonde entlang der Trajektorieachse von einem Basislinienpunkt der Trajektorieachse basierend auf den gefilterten Axialverschiebungsdaten; und
Modulieren des Alarms basierend auf dem bestimmten Abstand.

5. System nach Anspruch 4, wobei der Basislinienpunkt einer Position der Ablationssonde entspricht, wenn eine Ablationsprozedur initiiert wird; oder wobei der Basislinienpunkt durch einen Benutzer auswählbar ist.

6. Nicht-transitorisches computerlesbares Datenspeichermedium, das ausführbare Anweisungen speichert, die, wenn sie von einem Prozessor ausgeführt werden, eine Rechenvorrichtung hierzu veranlassen:
Empfangen von Positions- und Ausrichtungsdaten einer Ablationssonde (130) von einem Verfolgungssystem;
Anzeigen einer grafischen Darstellung der Ablationssonde auf einer Anzeige (110), basierend auf den empfangenen Positions- und Ausrichtungsdaten der Ablationssonde;
**dadurch gekennzeichnet, dass** die Anweisungen, wenn sie von dem Prozessor ausgeführt werden, den Prozessor zusätzlich hierzu veranlassen:
Filtern von Axialverschiebungsdaten aus den Positions- und Ausrichtungsdaten der Ablationssonde, wobei die gefilterten Axialverschiebungsdaten einer axialen Bewegung der Ablationssonde entlang einer Trajektorieachse entsprechen; und
Generieren eines Alarms basierend auf den gefilterten Axialverschiebungsdaten.

7. Nicht-transitorisches computerlesbares Datenspeichermedium nach Anspruch 6, wobei der Alarm wenigstens eines von einem akustischen Alarm oder einem visuellen Alarm ist.

8. Nicht-transitorisches computerlesbares Datenspeichermedium nach Anspruch 6 oder Anspruch 7, wobei die Anweisungen die Rechenvorrichtung ferner hierzu veranlassen:
Bestimmen einer Bewegungsgeschwindigkeit der Axialbewegung der Ablationssonde entlang der Trajektorieachse basierend auf den gefilterten Axialverschiebungsdaten; und
Modulieren des Alarms basierend auf der bestimmten Bewegungsgeschwindigkeit.

9. Nicht-transitorisches computerlesbares Datenspeichermedium nach einem der Ansprüche 6 bis 8, wobei die Anweisungen die Rechenvorrichtung ferner hierzu veranlassen:
Bestimmen eines Abstands der Axialbewegung der Ablationssonde entlang der Trajektorieachse ab einem Basislinienpunkt der Trajektorieachse basierend auf den gefilterten Axialverschiebungsdaten; und
Modulieren des Alarms basierend auf dem bestimmten Abstand.

10. Nicht-transitorisches computerlesbares Datenspeichermedium nach Anspruch 9, wobei der Basislinienpunkt einer Position der Ablationssonde entspricht, wenn eine Ablationsprozedur initiiert wird.

11. Nicht-transitorisches computerlesbares Datenspeichermedium nach Anspruch 9, wobei der Basislinienpunkt durch einen Benutzer auswählbar ist.

## Revendications

1. Système (10) permettant d'effectuer une procédure d'ablation par micro-ondes, le système comprenant :
une sonde d'ablation (130) configurée pour se coupler à un générateur électrochirurgical et comprenant un capteur électromagnétique ;
un système de suivi configuré pour suivre le mouvement de la sonde d'ablation dans un espace tridimensionnel et générer des données de position et d'orientation de la sonde d'ablation sur la base d'une position et d'une orientation du capteur électromagnétique ; et
un dispositif informatique (100) couplé de manière opérationnelle au système de suivi, le dispositif informatique comprenant un processeur (204) et une mémoire (202) stockant des instructions qui, lorsqu'elles sont exécutées par le processeur, amènent le dispositif informatique à :
recevoir les données de position et d'orientation de la sonde d'ablation en provenance du système de suivi ;
afficher une représentation graphique de la sonde d'ablation sur un écran (110) en fonction des données de position et d'orientation reçues de la sonde d'ablation ; **caractérisé en ce que** lesdites instructions, lorsqu'elles sont exécutées par le processeur, amènent en outre le dispositif informatique à :
filtrer des données de décalage axial à partir des données de position et d'orientation de la sonde d'ablation, les données de décalage axial filtrées correspondant à un mouvement axial de la sonde d'ablation le long d'un axe de trajectoire ; et
générer une alerte sur la base des données de décalage axial filtrées.

2. Système selon la revendication 1, dans lequel l'alerte est au moins une parmi une alerte sonore et une alerte visuelle.

3. Système selon la revendication 1 ou la revendication 2, dans lequel les instructions amènent en outre le dispositif informatique à :
déterminer une vitesse de mouvement du mouvement axial de la sonde d'ablation le long de l'axe de trajectoire sur la base des données de décalage axial filtrées ; et
moduler l'alerte en fonction de la vitesse de mouvement déterminée.

4. Système selon une quelconque revendication précédente, dans lequel les instructions amènent en outre le dispositif informatique à :
déterminer une distance du mouvement axial de la sonde d'ablation le long de l'axe de trajectoire à partir d'un point de référence sur l'axe de trajectoire sur la base des données de décalage axial filtrées ; et
moduler l'alerte en fonction de la distance déterminée.

5. Système selon la revendication 4, dans lequel le point de référence correspond à un emplacement de la sonde d'ablation lorsqu'une procédure d'ablation est lancée ; ou dans lequel le point de référence peut être sélectionné par un utilisateur.

6. Support de stockage non transitoire lisible par ordinateur stockant des instructions qui, lorsqu'elles sont exécutées par un processeur, amènent un dispositif informatique à :
recevoir des données de position et d'orientation d'une sonde d'ablation (130) en provenance d'un système de suivi ;
afficher une représentation graphique de la sonde d'ablation sur un écran (110) en fonction des données de position et d'orientation reçues de la sonde d'ablation ; **caractérisé en ce que** lesdites instructions, lorsqu'elles sont exécutées par le processeur, amènent en outre le dispositif informatique à :
filtrer des données de décalage axial à partir des données de position et d'orientation de la sonde d'ablation, les données de décalage axial filtrées correspondant à un mouvement axial de la sonde d'ablation le long d'un axe de trajectoire ; et
générer une alerte sur la base des données de décalage axial filtrées.

7. Support de stockage non transitoire lisible par ordinateur selon la revendication 6, dans lequel l'alerte est au moins l'une d'une alerte sonore et d'une alerte visuelle.

8. Support de stockage non transitoire lisible par ordinateur selon la revendication 6 ou la revendication 7, dans lequel les instructions amènent en outre le dispositif informatique à :
déterminer une vitesse de mouvement du mouvement axial de la sonde d'ablation le long de l'axe de trajectoire sur la base des données de décalage axial filtrées ; et
moduler l'alerte en fonction de la vitesse de mouvement déterminée.

9. Support de stockage non transitoire lisible par ordinateur selon l'une quelconque des revendications 6 à 8, dans lequel les instructions amènent en outre le dispositif informatique à :
déterminer une distance du mouvement axial de la sonde d'ablation le long de l'axe de trajectoire à partir d'un point de référence sur l'axe de trajectoire sur la base des données de décalage axial filtrées ; et
moduler l'alerte en fonction de la distance déterminée.

10. Support de stockage non transitoire lisible par ordinateur selon la revendication 9, dans lequel le point de référence correspond à un emplacement de la sonde d'ablation lorsqu'une procédure d'ablation est lancée.

11. Support de stockage non transitoire lisible par ordinateur selon la revendication 9, dans lequel le point de référence peut être sélectionné par un utilisateur.
